# EUROPÄISCHE PATENTSCHRIFT

(11) **EP 3 271 710 B1**
(45) Veröffentlichungstag und Bekanntmachung des Hinweises auf die Patenterteilung: **29.04.2020**
(21) Anmeldenummer: 16718993.5
(22) Anmeldetag: 17.03.2016
(51) Int. Cl.: G01N 27/22, G01N 33/483

(54) **ANORDNUNG ZUR BESTIMMUNG DER FEUCHTIGKEIT EINES GEGENSTANDES**
ARRANGEMENT FOR MEASURING THE HUMIDITY OF AN OBJECT
ENSEMBLE POUR DÉTERMINER L'HUMIDITÉ D'UN OBJET

(30) Priorität: 20.03.2015 AT 502262015
(43) Veröffentlichungstag der Anmeldung: 24.01.2018
(73) Patentinhaber: AIT Austrian Institute of Technology GmbH, 1220 Wien (AT)
(72) Erfinder: OBERLEITNER, Andreas, 2492 Zillingdorf (AT); LURF, Robert, 2640 Gloggnitz (AT)
(74) Vertreter: Wildhack & Jellinek
(86) Internationale Anmeldenummer: PCT/AT2016/050066
(87) Internationale Veröffentlichungsnummer: WO 2016/149720

(56) Entgegenhaltungen:
- EP-A1- 2 500 094
- WO-A1-2012/140310
- UNANDER T ET AL: "Characterization of Printed Moisture Sensors in Packaging Surveillance Applications", IEEE SENSORS JOURNAL, IEEE SERVICE CENTER, NEW YORK, NY, US, Bd. 8, Nr. 8, 26. Juni 2009 (2009-06-26), Seiten 922-928, XP011263826, ISSN: 1530-437X
- FANGMING DENG ET AL: "A CMOS Humidity Sensor for Passive RFID Sensing Applications", SENSORS, Bd. 14, Nr. 5, 16. Mai 2014 (2014-05-16), Seiten 8728-8739, XP055280944, DOI: 10.3390/s140508728
- ROSE DANIEL P ET AL: "Adhesive RFID Sensor Patch for Monitoring of Sweat Electrolytes", IEEE TRANSACTIONS ON BIOMEDICAL ENGINEERING, IEEE SERVICE CENTER, PISCATAWAY, NJ, USA, Bd. 62, Nr. 6, 11. November 2014 (2014-11-11), Seiten 1457-1465, XP011581742, ISSN: 0018-9294, DOI: 10.1109/TBME.2014.2369991 [gefunden am 2015-05-18]
- "GoSense TH-Stat ID(TM) NFC Temp & Humidity Sensor", , 1. Mai 2013 (2013-05-01), Seiten 1-1, XP055228577, Gefunden im Internet: URL:http://www.gosense-wireless.com/GoSens e TH-StatID DS Rev1.pdf [gefunden am 2015-11-16]
- David Wang: "FDC1004: Basics of Capacitive Sensing and Applications", Application Report, 1. Dezember 2014 (2014-12-01), Seiten 1-12, XP055279836, Gefunden im Internet: URL:http://www.ti.com/lit/an/snoa927/snoa9 27.pdf [gefunden am 2016-06-13]

## Beschreibung

Die Erfindung betrifft eine Anordnung zur Bestimmung der Feuchtigkeit eines Gegenstands, insbesondere der Feuchtigkeit der menschlichen Haut.

Die WO 2012/140310 A1 zeigt einen Sensor mit einem RFID-Transponder und einem Kondensator zur Bestimmung unterschiedlicher Umgebungsparameter wie z. B. der Feuchtigkeit. Unander, T. et al.: "Characterization of Printed Moisture Sensors in Packaging Surveillance Applications", IEEE Sensors Journal, IEEE Service Center, New York, NY, US, Bd. 8, Nr. 8, 26. Juni 2009, zeigt mit leitfähiger Tinte auf Papiersubstrate aufdruckbare Feuchtigkeitssensoren für Verpackungen. Die EP 2 500 094 A1 offenbart Feuchtigkeitssensoren mit einer semi-permeablen Membran für NFC-Anordnungen für Verpackungen. Fangming Deng et al.: "A CMOS Humidity Sensor for Passive RFID Sensing Applications", Sensors, Bd. 14, Nr. 5, 16. Mai 2014, offenbart einen Feuchtigkeitssensor für Anwendungen, die passive RFID-Technologie implementieren.

Aus dem Stand der Technik sind eine Vielzahl von Hautfeuchtigkeitsmessgeräten bekannt, die allesamt gewisse Nachteile aufweisen. Insbesondere ist es schwierig, bei solchen Feuchtigkeitsmessgeräten auf einfache Weise berührungslos unverfälschte Messwerte jeweils von derselben Stelle der Haut zu erlangen. Zudem ist es bei aus dem Stand der Technik bekannten Tischgeräten zwar möglich, unter Laborbedingungen zu messen, diese sind aber nicht mobil. Bei Impedanzverfahren ist galvanischer Hautkontakt und damit eine Spannungsversorgung erforderlich. Die ermittelten Daten sind nicht mobil digital verfügbar oder weiterverarbeitbar.

Aufgabe der Erfindung ist es, diese Probleme zu lösen.

Die Erfindung löst diese Aufgabe mit einer Anordnung mit den kennzeichnenden Merkmalen des Patentanspruchs 1.

Die Erfindung betrifft eine Anordnung zur Bestimmung der Feuchtigkeit eines Gegenstands, insbesondere der Feuchtigkeit der menschlichen Haut, umfassend eine elektrisch isolierende Trägerschicht, auf der die folgenden Einheiten aufgebracht sind:
- zumindest zwei Elektroden, insbesondere Interdigitalelektroden, die auf einer ersten Seite der Trägerschicht angeordnet sind,
- eine digitale Kapazitätsmesseinrichtung, an die die Elektroden angeschlossen sind,
- einen NFC-Transponder mit einer Antenne, dem die Messergebnisse der Kapazitätsmesseinrichtung zur Weiterleitung mittels Funk über die Antenne zugeführt sind, wobei der NFC-Transponder und die Kapazitätsmesseinrichtung insbesondere im selben Chip angeordnet sind, und

- eine Schirmung, die auf der der ersten Seite gegenüberliegenden Seite der Trägerschicht liegt und insbesondere mit der Systemmasse der Kapazitätsmesseinrichtung verbunden ist.

Erfindungsgemäß ist dabei vorgesehen, dass
- auf derjenigen Seite der Trägerschicht, die den Elektroden gegenüberliegt, ein Entlastungselement vorgesehen ist, das von der Trägerschicht absteht und innerhalb eines Randbereichs um die Elektroden angeordnet ist, dessen Breite größer ist als 15% der längsten Abmessung des von den Elektroden umfassten Flächenbereichs und
- das Entlastungselement eine Dicke von zwischen 0,5 mm und 2 mm aufweist.

Diese Ausgestaltung der Anordnung dient zur Verringerung des Einflusses mechanischer Belastungen auf das Messergebnis

Der mechanisch einfache und stabile Aufbau verringert den Einfluss mechanischer Belastungen auf das Messergebnis weiter.

Eine solche Anordnung hat zudem den Vorteil, dass sie massentauglich ist und günstig herstellbar ist sowie eine einfache Anwendung im Hinblick auf das Auslesen der ermittelten Gegenstands- oder Hautfeuchtigkeitsmesswerte ermöglicht. Weiters ist aufgrund der Berührungslosigkeit der Initiierung des Messens nur eine geringe Anpressdruckabhängigkeit der Messwerte gegeben. Dies ist speziell bei kompressiblen Gegenständen, wie beispielsweise Haut, wichtig. Daraus resultiert eine geringe Beeinflussung des Messwerts, da die Messung auf Anfrage eines externen Datenkommunikationsgeräts insgesamt berührungslos erfolgt.

Um eine ortsfeste Messung insbesondere jeweils an der gleichen Stelle des menschlichen Körpers zu ermitteln und eine Vielzahl mehrerer Messungen jeweils an derselben Stelle vornehmen zu können, kann vorgesehen sein, dass eine Isolationsschicht vorgesehen ist, die auf derjenigen Seite der Trägerschicht angeordnet ist, die der Schirmung gegenüberliegt und auf den Elektroden aufliegt,
wobei insbesondere die Isolationsschicht an der den Elektroden abgewandten Seite klebend ausgebildet ist.

Eine einfach zu fertigende Ausführungsform der Erfindung kann erreicht werden, indem , die Isolationsschicht eine Folie aufweist, an deren den Elektroden zugewandten Seite eine erste Verbindungsschicht, insbesondere Klebeschicht, angeordnet ist, die die Folie, insbesondere klebend, mit den Elektroden und/oder der Trägerschicht verbindet, und
an der anderen Seite der Folie, die den Elektroden abgewandt ist, eine zweite Klebeschicht zum Anhaften an den Gegenstand vorhanden ist.

Um den Einfluss der Trägerschicht und Klebeschicht auf die Messung zu verringern und einen größeren Einfluss der eigentlichen Messgröße, nämlich der (Haut)feuchtigkeit, auf die gemessene Kapazität zu erreichen und parasitäre Wirkungen zu verringern, kann vorgesehen sein, dass die Trägerschicht und/oder die Isolationsschicht eine relative Permittivität von weniger als 20, insbesondere von weniger als 5, aufweisen.

Um den manuellen Zugriff auf die Schirmung bzw. die elektronischen Bauteile zu verhindern und die elektronischen Bauteile gegen Feuchte und mechanische Einflüsse zu schützen, kann vorgesehen sein, dass auf der Seite der Trägerschicht, an der sich die Schirmung befindet, eine Deckschicht angeordnet ist.

Um die Beeinflussung durch externe Felder zu verringern und die Schirmung zu verbessern, kann vorgesehen sein, dass die Deckschicht elektrisch isolierend ausgebildet ist und die Schirmung vor Zugriffen von außen elektrisch isoliert.

Eine besonders einfache Herstellung der Antenne mittels gedruckter Schaltungen kann erreicht werden, indem die Windungen der Antenne als leitfähige Schicht auf einer Seite der Trägerschicht aufgebracht und angeordnet sind.

Um eine Anpassung der Anordnung an nicht planare Oberflächen, wie sie auf der menschlichen Haut typisch sind, zu erreichen, kann vorgesehen sein, dass die Trägerschicht und/oder die Isolierschicht und/oder die Abdeckschicht einen flexiblen Aufbau aufweisen.

Um eine Aufzeichnung von Datenwerten ohne Interaktion des Anwenders zu erreichen und diese Datenwerte zeitversetzt auszulesen, kann vorgesehen sein, eine Batterie vorhanden ist, die an den Kapazitätsmesseinrichtung angeschlossen ist, und insbesondere
die Kapazitätsmesseinrichtung zu vorgegebenen Zeitpunkten die Kapazität zwischen den Elektroden misst und diese in einem Datenzwischenspeicher abspeichert, wobei der NFC-Transponder sämtliche im Datenzwischenspeicher befindlichen Kapazitätsmesswerte auf Anfrage überträgt.

Alternativ ist es auch möglich, dass der NFC-Transponder die Kapazitätsmesseinrichtung mit in einem Energiezwischenspeicher abgespeicherten Energie, die aus dem die Antenne umgebenden elektromagnetischen Feld entnommen wurde, versorgt.

Zur Verringerung des Einflusses mechanischer Belastungen auf das Messergebnis kann vorgesehen sein, dass auf der Trägerschicht, insbesondere auf allen Seiten, ein Rand vorgesehen ist, der frei von Elektroden ist, wobei die Breite des Randes der Trägerschicht dabei zumindest 15%, insbesondere zumindest 30% der längsten Abmessung des von den Elektroden beanspruchten Flächenbereichs misst.

Bevorzugte Ausführungsformen der Erfindung werden anhand der im Folgenden dargestellten Zeichnungsfiguren näher erörtert.

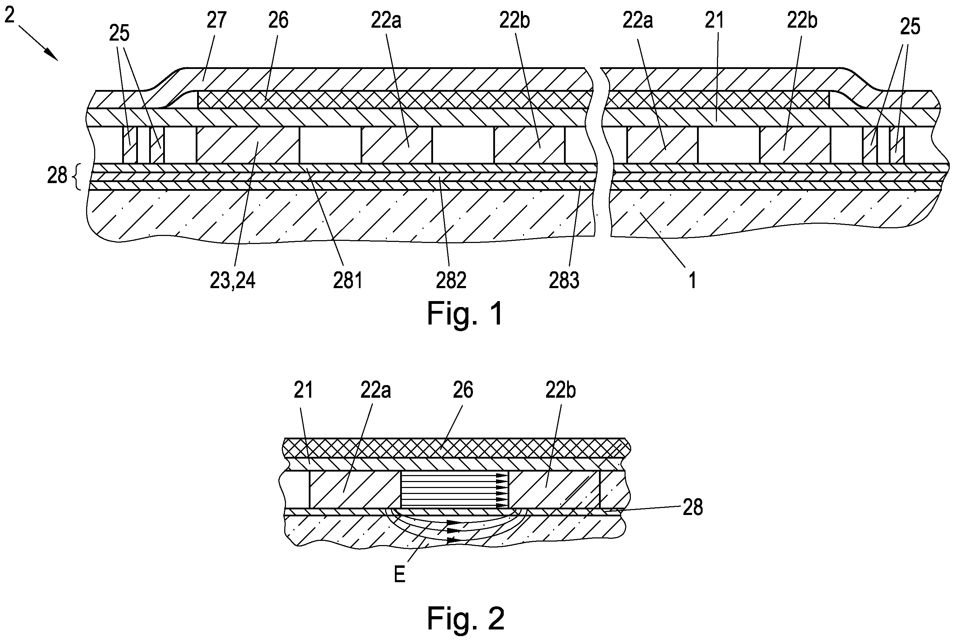

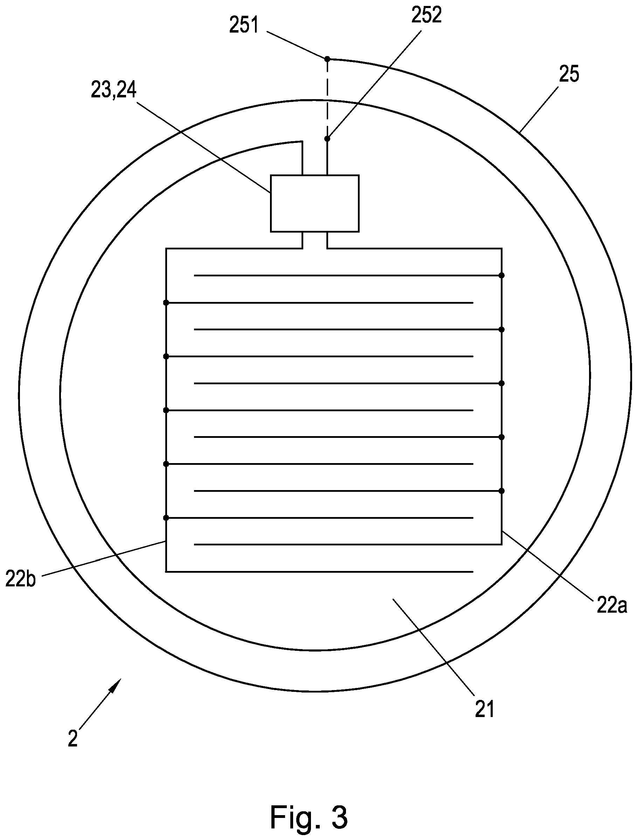

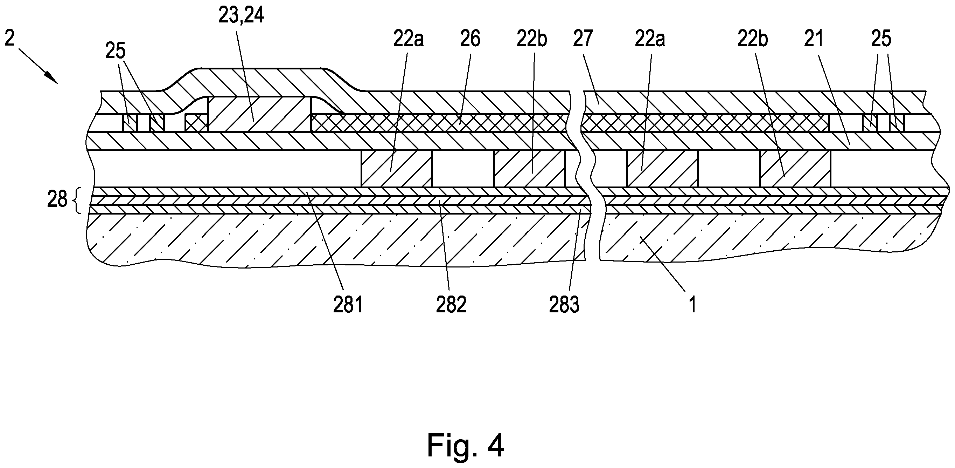

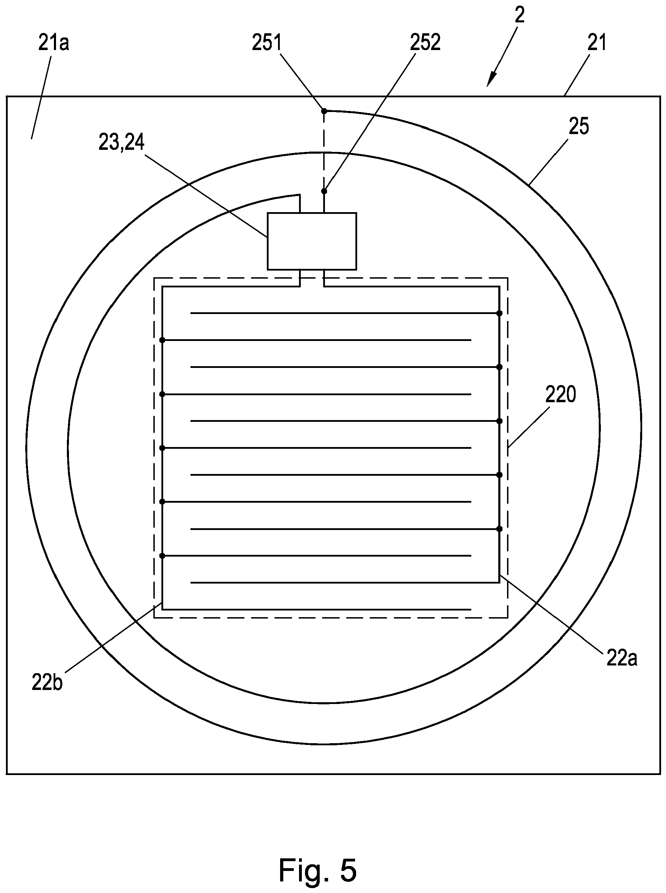

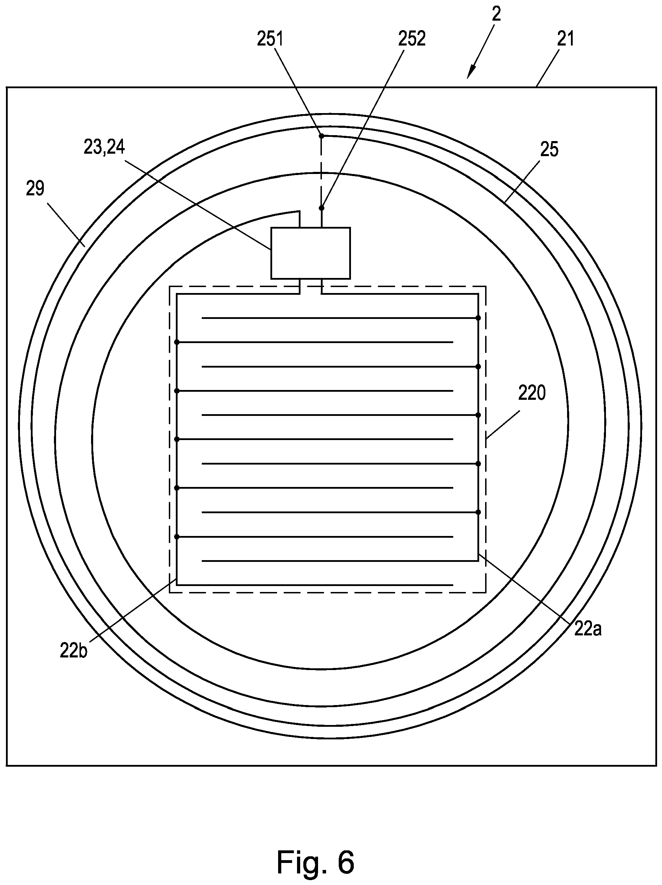

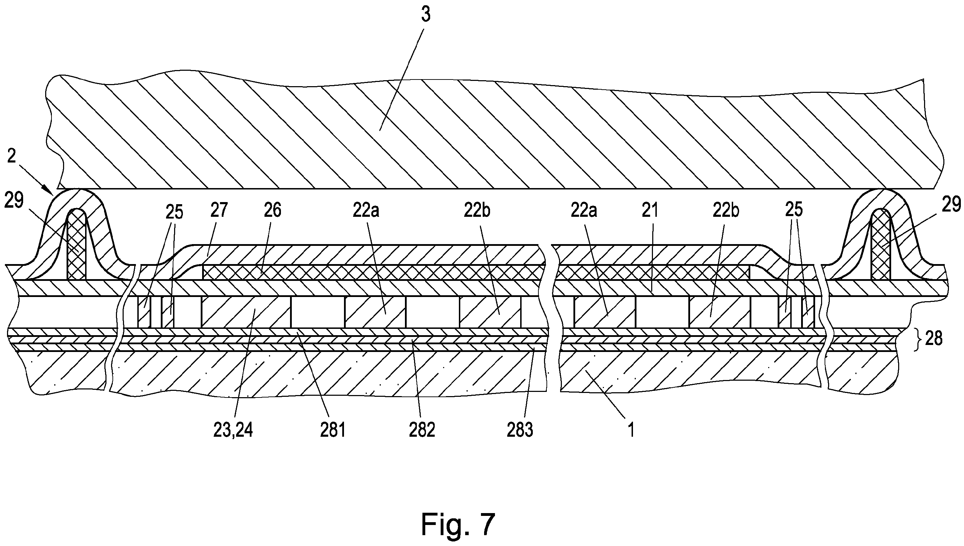

Fig. 1 zeigt im Schnitt eine Anordnung gemäß eines ersten Beispiels. **Fig. 2** zeigt im Detail die elektrischen Verhältnisse zwischen zwei Elektroden, die an die menschliche Haut angelegt sind. **Fig. 3** zeigt die in **Fig. 1** dargestellte Anordnung von dem zu untersuchenden Gegenstand aus gesehen. **Fig. 4** zeigt ein alternatives Beispiel einer Anordnung, bei der die Kapazitätsmesseinrichtung und der NFC-Transponder auf der den Elektroden gegenüberliegenden Seite der Trägerschicht angeordnet sind. **Fig. 5** zeigt ein weiteres Beispiel mit einem im Träger vorgesehenen Rand. **Fig. 6** **und** **7** zeigen eine Ausführungsform der Erfindung mit einem auf dem Träger angeordneten Entlastungselement.

Die in **Fig. 1** dargestellte Anordnung 2 zur Bestimmung der Feuchtigkeit eines Gegenstands 1 weist eine elektrisch isolierende Trägerschicht 21 auf. An einer Seite der elektrisch isolierenden Trägerschicht 21 befinden sich zwei Elektroden 22a, 22b, die, wie in **Fig. 3** dargestellt, als Interdigitalelektroden ausgebildet sind. Weiters ist in **Fig. 1** eine digitale Kapazitätsmesseinrichtung 23 dargestellt, an die die Elektroden 22a, 22b angeschlossen sind (**Fig. 3**). Im selben Chip wie die Kapazitätsmesseinrichtung 23 ist auch ein NFC-Transponder 24 angeordnet, der an eine Antenne 25 angeschlossen ist, die im Umfangsbereich der Anordnung 2 angeordnet ist. Die Kapazitätsmesseinrichtung 23 sowie der NFC-Transponder 24 können auch in getrennten Chips angeordnet sein.

Dem NFC-Transponder 24 sind die Messergebnisse der Kapazitätsmesseinrichtung 23 zugeführt. Gelangt eine Anfrage von einem externen Datenkommunikationsgerät über die Antenne 25 an den NFC-Transponder 24, so triggert dieser die Kapazitätsmesseinrichtung 23 zur Messung der Kapazität an ihrem Eingang, das heißt zwischen den an sie angeschlossenen Elektroden 22a, 22b. Die Kapazitätsmesseinrichtung 23 misst die zwischen den Elektroden 22a, 22b vorgegebene Kapazität und leitet das Messergebnis an den NFC-Transponder 24 weiter, der das Messergebnis über die Antenne 25 an das externe Datenkommunikationsgerät übermittelt. Zur Energieversorgung des NFC-Transponders 24 und der Kapazitätsmesseinrichtung 23 wird üblicherweise über die Antenne 25 vom NFC-Transponder 24 Energie aus dem Feld des externen Datenkommunikationsgeräts entzogen und vom NFC-Transponder 24 oder von der Kapazitätsmesseinrichtung 23 in einem dafür vorgesehenen Energiezwischenspeicher zwischengespeichert. Dabei wird dem die Antenne 25 umgebenden Feld so viel Energie entzogen und im Energiezwischenspeicher zwischengespeichert, dass der jeweils durchzuführende Messvorgang abgeschlossen werden kann.

Weiters weist die Anordnung 2 eine Schirmung 26 auf, die an der der ersten Seite gegenüberliegenden Seite, das heißt auf der den Elektroden gegenüberliegenden Seite der Trägerschicht, liegt. Im vorliegenden Beispiel ist die Schirmung mit der Systemmasse der Kapazitätsmesseinrichtung 23 verbunden. Es bestehen aber auch andere Möglichkeiten, beispielsweise kann die Schirmung auch an das aktive Potential angeschlossen werden oder bei entsprechend größerer Ausführung auch überhaupt unkontaktiert bleiben.

Alternativ kann der Schirm nicht durchgängig sein, sondern exakt über der Kontur der Elektroden liegen. Somit ergeben sich zwei unterschiedliche, voneinander getrennte Schirmungen.

Weiters umfasst dieses Beispiel auch eine Isolationsschicht 28, die auf derjenigen Seite der Trägerschicht angeordnet ist, die der Schirmung 26 gegenüber liegt bzw. an den Elektroden 22a, 22b anliegt oder auf diesen aufliegt. Die Isolationsschicht 28 ist an der den Elektroden 22a, 22b abgewandten Seite klebend ausgebildet. Wie sich weiter aus Fig. 1 ergibt, besteht die Isolationsschicht 28 aus drei Teilschichten, nämlich einer zentralen Folie 282 sowie einer ersten Klebeschicht 281, die zwischen der Folie und den Elektroden 22a, 22b liegt und einer zweiten Klebeschicht 283, die dem Gegenstand 1, insbesondere der zu untersuchenden menschlichen Haut, zugewandt ist. Daneben bestehen auch zahlreiche andere Möglichkeiten, eine Isolationsschicht herzustellen, etwa als Laminatverbund.

In **Fig. 2** ist schematisch die Kapazitätsmessung zwischen den beiden Elektroden 22a, 22b dargestellt. Zwischen den beiden Elektroden 22a, 22b wird ein elektrisches Wechselfeld angelegt, das im vorliegenden Beispiel eine Frequenz von etwa 100 kHz aufweist. Grundsätzlich können zur Messung der Leitfähigkeit der Haut Frequenzen von etwa 40 kHz bis etwa 500 kHz verwendet werden.

Eine Möglichkeit der Kapazitätsmessung kann vorgenommen werden, indem die integrierte Kapazitätsmesseinrichtung 23 ein Sigma-Delta Verfahren ausführt, bei welchem der unbekannte Kondensator mit einer fixen Spannung gepulst wird. Die Anzahl der Pulse, die notwendig ist, um eine fixe Referenz zu erreichen, erlaubt Rückschlüsse auf die Kapazität.

Im Zwischenbereich zwischen den beiden Elektroden 22a, 22b bildet sich eine Kapazität aus, die für die vorliegende Messung als parasitär betrachtet werden kann und die von der eigentlichen Messgröße, nämlich der Feuchte des Gegenstands, insbesondere der Haut, im obersten Bereich des Gegenstands 1 nicht beeinflusst ist. Das von den beiden Elektroden 22a, 22b ausgehende elektrische Feld dringt durch die Isolationsschicht 28 hindurch in den Gegenstand 1 bzw. in dessen obere Bereiche ein. Hierdurch wird die zwischen den beiden Elektroden gemessene Kapazität abhängig vom Wassergehalt in den jeweils oberen Hautschichten. Um eine Beeinflussung der Messung durch die Trägerschicht oder die Klebeschicht zu vermeiden, werden als Materialien für die Trägerschicht 21 und die Isolationsschicht 28 Materialien mit einer geringen relativen Permittivität von weniger als 20, insbesondere von weniger als 5, verwendet. Typische Materialien für die Trägerschicht sind etwa Kunststoffe, wie PET oder Polyimid, bzw. Polyesterfolien; typische Materialien für Klebstoffe wären z.B. Acrylatpolymere.

Die Trägerschicht 21, die Isolationsschicht 28 und die Abdeckschicht 27 sind grundsätzlich als flexible und flache Schichten ausgebildet, um eine Anpassung an nicht planare Oberflächen zu ermöglichen.

Zum Schutz der verwendeten elektronischen Komponenten auf der Trägerschicht 21 ist auf der Seite der Trägerschicht 21, an der sich die Schirmung 26 befindet, eine Deckschicht 27 angeordnet, die sämtliche elektronischen Komponenten, insbesondere die Schirmung abdeckt. Die Deckschicht 27 ist elektrisch isolierend ausgebildet und isoliert die Schirmung 26 vor Zugriffen von außen, hierdurch wird auch ein auch mechanischer Basisschutz gewährleistet. In dem alternativen Beispiel der **Fig. 4** sind neben der Abschirmung 26 auch der Chip umfassend den NFC-Transponder 24 und die Kapazitätsmesseinrichtung 23 sowie die Antenne 25 auf der den Elektroden 22a, 22b gegenüberliegenden Seite der Trägerschicht 21 angeordnet. Auch diese Komponenten werden, wie in **Fig. 4** dargestellt, von der Deckschicht 27 vor Zugriffen von außen geschützt.

Sowohl bei dem in **Fig. 1** als auch bei dem in **Fig. 4** darstellten Beispiel ist die Antenne als leitfähige Schicht ausgebildet, die vollständig auf einer Seite der Trägerschicht 21 aufgebracht oder angeordnet ist. Bei dem in **Fig. 1** dargestellten Beispiel ist die Antenne als Schicht auf derselben Seite wie die Elektroden 22a, 22b angeordnet. Insbesondere können die Elektroden wie auch die Antenne 25 als gedruckte Schaltung auf der Trägerschicht 21 angeordnet sein. Wie aus **Fig. 3** ersichtlich, ist die Antenne bei dem in **Fig. 1** dargestellten Beispiel in einem Überkreuzungsbereich zwischen den Punkten 251, 252 an der den Elektroden abgewandten Seite der Trägerschicht geführt.

Bei sämtlichen Beispielen und Ausführungsformen der Erfindung ist es möglich, anstelle oder zusätzlich zur Entnahme der Energie aus dem elektromagnetischen Feld mittels der Antenne auch eine in den Fig. nicht dargestellte Batterie vorzusehen, an die die Kapazitätsmesseinrichtung 23 und gegebenenfalls auch der NFC-Transponder 24 angeschlossen sind. Hierbei besteht die Möglichkeit, dass die Kapazitätsmesseinrichtung 23 zu automatisch vorgegebenen Zeitpunkten die Kapazität zwischen den Elektroden 22a, 22b misst und diese in einem Datenzwischenspeicher abspeichert, der ebenfalls im Chip gemeinsam mit dem NFC-Transponder 24 und der Kapazitätsmesseinrichtung 23 integriert sein kann. Auf Anfrage eines externen Datenkommunikationsgeräts besteht die Möglichkeit, dass sämtliche im Datenzwischenspeicher abgespeicherten Kapazitätsmesswerte vom NFC-Transponder 24 an das externe Datenkommunikationsgerät übertragen werden.

Die beiden im Folgenden dargestellten Ausführungsformen der Erfindung sind geeignet, die Einflüsse von unkonstantem Anpressdruck auf die Messergebnisse zu unterdrücken bzw zu minimieren.

In **Fig. 5** ist ein weiteres Beispiel dargestellt, das bis auf die im Folgenden dargestellten Details den in **Fig. 1** oder **Fig. 4** dargestellten Beispielen entspricht. Bei diesem Beispiel ist auf allen Seiten der Trägerschicht 21 ein Rand 21a vorgesehen, der frei von Elektroden 22a, 22b ist. Die Breite des Randes 21a der Trägerschicht 21 misst dabei zumindest 15%, insbesondere zumindest 30% der längsten Abmessung des von den Elektroden 22a, 22b beanspruchten Flächenbereichs 220. Ist der die Elektroden 22a, 22b tragende Flächenbereich 220 wie in dem in **Fig. 5** dargestellten Beispiel rechteckig ausgebildet, so kann die Trägerschicht 21 ebenfalls rechteckig ausgebildet sein, wobei ein umlaufender Rand 21a vorgesehen ist. Die Breite des Rands 21a entspricht in dem dargestellten Beispiel 50% der jeweiligen Kantenlänge des rechteckigen Flächenbereichs 220. Um signifikante Verbesserungen der Messung zu erzielen, reicht mitunter auch ein Rand 21a mit einer Breite von mindestens 15% der jeweiligen Kantenlänge des rechteckigen Flächenbereichs 220.

In **Fig. 6** **und** **7** ist eine bevorzugte Ausführungsform einer erfindungsgemäßen Anordnung dargestellt, die bis auf die im Folgenden dargestellten Details dem in **Fig. 1** **und** **4** dargestellten Beispiel entspricht.

Auf derjenigen Seite des Trägers 21, die dem Gegenstand 1 abgewandt ist bzw die den Elektroden 22a, 22b gegenüber liegt, befindet sich ein Entlastungselement 29, das in der vorliegenden Ausführungsform als Entlastungsring 29 ausgebildet ist. Das Entlastungselement 29 umgibt die Elektroden 22a, 22b derart, dass bei Auflage eines flachen Gegenstands 3 wie insbesondere eines Lesegeräts 3, insbesondere in Form eines Mobiltelefons 3, auf dem Entlastungselement 29, Auflagekräfte nicht auf die Elektroden 22a, 22b wirken und damit keine Verzerrung des Messergebnisses bewirken. Das Entlastungselement 29 hält das Lesegerät 3 in ausrechendem Abstand zu den Elektroden 22a, 22b des Trägers 21. Auch die vom Lesegerät 3 eingebrachten Kräfte wirken im ausreichenden Abstand von den Elektroden 22a, 22b auf den Träger ein, um Verzerrungen des Trägers 21 im Bereich dieser Elektroden 22a, 22b zu vermeiden und den Träger 21 im Bereich der Elektroden 22a, 22b von Spannungen und Verzerrungen zu entlasten.

Die Geometrie des Entlastungselements 29 ermöglicht, wie in **Fig. 7** dargestellt, eine Ableitung der Auflagekräfte in den Gegenstand 1 bzw den menschlichen Körper an den Elektroden 22a, 22b vorbei. Um dies zu gewährleisten, können grundsätzlich unterschiedliche Ausführungsformen eines Entlastungselements 29 gewählt werden. In einer bevorzugten Variante ist der Entlastungselement 29 aus flexiblem Gummimaterial ausgebildet.

Das Entlastungselement 29 ist erfindungsgemäß zwischen 0,5mm und 2mm dick. Dies hat den Vorteil, dass das Entlastungselement 29 mit dieser Dicke noch einfach in den Träger 21 integrierbar ist und andererseits das Lesegerät 3 ausreichend auf Abstand hält, um Verzerrungen des Trägers im Bereich der Elektroden 22a, 22b zu vermeiden und den Träger 21 im Bereich der Elektroden 22a, 22b zu entlasten.

Das Entlastungselement 29 ist vorteilhafterweise innerhalb eines Randbereichs 21a um die Elektroden 22a, 22b angeordnet, dessen Breite größer ist als 15% der längsten Abmessung des von den Elektroden 22a, 22b beanspruchten Flächenbereichs 220. Durch diesen Abstand wird vermieden, dass über das Lesegerät oder sonst ausgeübte Kräfte in den Bereich der Elektroden 22a, 22b übertragen werden und derart die Messung verfälscht wird.

Im vorliegenden Anwendungsbeispiel liegt diese Breite zwischen 15% der längsten Abmessung des von den Elektroden 22a, 22b beanspruchten Flächenbereichs 220 und 200 mm. Diese Breite liegt zwischen 15% und 200% der längsten Abmessung des von den Elektroden 22a, 22b beanspruchten Flächenbereichs 220.

Das Entlastungselement kann sowohl unter der Deckschicht 27 als auch über der Deckschicht 27 platziert werden.

Bei allen Ausführungsformen können die Trägerschicht 21 und/oder die Isolationsschicht 28 atmungsaktiv ausgebildet sein, um Schweiß- und Flüssigkeitsansammlungen zu vermeiden.

## Patentansprüche

1. Anordnung (2) zur Bestimmung der Feuchtigkeit eines Gegenstands (1) umfassend eine elektrisch isolierende Trägerschicht (21), auf der die folgenden Einheiten aufgebracht sind:
- zumindest zwei Elektroden (22a, 22b) die auf einer ersten Seite der Trägerschicht (21) angeordnet sind,
- eine digitale Kapazitätsmesseinrichtung (23), an die die Elektroden (22a, 22b) angeschlossen sind,
- einen NFC-Transponder (24) mit einer Antenne (25), dem die Messergebnisse der Kapazitätsmesseinrichtung (23) zur Weiterleitung mittels Funk über die Antenne (25) zugeführt sind,
- eine Schirmung (26), die auf der der ersten Seite gegenüberliegenden Seite der Trägerschicht (21) liegt,
**dadurch gekennzeichnet, dass**
- auf derjenigen Seite der Trägerschicht (21), die den Elektroden (22a, 22b) gegenüberliegt, ein Entlastungselement (29) vorgesehen ist, das von der Trägerschicht (21) absteht und innerhalb eines Randbereichs (21a) um die Elektroden (22a, 22b) angeordnet ist, dessen Breite größer ist als 15% der längsten Abmessung des von den Elektroden (22a, 22b) umfassten Flächenbereichs (220) und
- das Entlastungselement (29) eine Dicke von zwischen 0,5 mm und 2 mm aufweist.

2. Anordnung (2) nach Anspruch 1, ferner umfassend eine Isolationsschicht (28), die auf derjenigen Seite der Trägerschicht (21) angeordnet ist, die der Schirmung (26) gegenüberliegt und auf den Elektroden (22a, 22b) aufliegt,
wobei insbesondere die Isolationsschicht (28) an der den Elektroden (22a, 22b) abgewandten Seite klebend ausgebildet ist.

3. Anordnung (2) nach Anspruch 2, **dadurch gekennzeichnet, dass** die Isolationsschicht (28) eine Folie (282) aufweist, an deren den Elektroden (22a, 22b) zugewandten Seite eine erste Verbindungsschicht, insbesondere Klebeschicht (281), angeordnet ist, die die Folie (282), insbesondere klebend, mit den Elektroden (22a, 22b) und/oder der Trägerschicht (21) verbindet, und
dass an der anderen Seite der Folie (28), die den Elektroden (22a, 22b) abgewandt ist, eine zweite Klebeschicht (283) zum Anhaften an den Gegenstand (1) vorhanden ist.

4. Anordnung (2) nach einem der vorangehenden Ansprüche, **dadurch gekennzeichnet, dass** die Trägerschicht (21) und/oder die Isolationsschicht (28) eine relative Permittivität von weniger als 20, insbesondere von weniger als 5, aufweisen.

5. Anordnung (2) nach einem der vorangehenden Ansprüche, **dadurch gekennzeichnet, dass** auf der Seite der Trägerschicht (21), an der sich die Schirmung (26) befindet, eine Deckschicht (27) angeordnet ist.

6. Anordnung (2) nach Anspruch 5, **dadurch gekennzeichnet, dass** die Deckschicht (27) elektrisch isolierend ausgebildet ist und die Schirmung (26) vor Zugriffen von außen elektrisch isoliert.

7. Anordnung (2) nach einem der vorangehenden Ansprüche, **dadurch gekennzeichnet, dass** die Windungen der Antenne (25) als leitfähige Schicht auf einer Seite der Trägerschicht (21) aufgebracht und angeordnet sind.

8. Anordnung (2) nach einem der vorangehenden Ansprüche, **dadurch gekennzeichnet, dass** die Trägerschicht (21) und/oder die Isolierschicht (28) und/oder die Abdeckschicht (27) einen flexiblen Aufbau aufweisen.

9. Anordnung (2) nach einem der vorangehenden Ansprüche, **dadurch gekennzeichnet, dass** eine Batterie vorhanden ist, die an der Kapazitätsmesseinrichtung (23) angeschlossen ist, und insbesondere
die Kapazitätsmesseinrichtung (23) eingerichtet ist, zu vorgegebenen Zeitpunkten die Kapazität zwischen den Elektroden (22a, 22b) zu messen und diese in einem Datenzwischenspeicher abzuspeichern, wobei der NFC-Transponder (24) eingerichtet ist, sämtliche im Datenzwischenspeicher befindlichen Kapazitätsmesswerte auf Anfrage zu übertragen.

10. Anordnung (2) nach einem der vorangehenden Ansprüche, **dadurch gekennzeichnet, dass** der NFC-Transponder (24) die Kapazitätsmesseinrichtung (23) mit in einem Energiezwischenspeicher abgespeicherter Energie, die aus dem die Antenne (25) umgebenden elektromagnetischen Feld entnommen wurde, versorgt.

11. Anordnung nach einem der vorangehenden Ansprüche, **dadurch gekennzeichnet, dass** auf der Trägerschicht (21), insbesondere auf allen Seiten, ein Rand (21a) vorgesehen, der frei von Elektroden (22a, 22b) ist, wobei die Breite des Randes (21a) der Trägerschicht (21) dabei zumindest 15%, insbesondere zumindest 30% der längsten Abmessung des von den Elektroden (22a, 22b) beanspruchten Flächenbereichs (220) misst.

12. Anordnung nach einem der vorangehenden Ansprüche, **dadurch gekennzeichnet, dass** die Anordnung zur Bestimmung der Feuchtigkeit der menschlichen Haut ausgebildet ist.

13. Anordnung nach einem der vorangehenden Ansprüche, **dadurch gekennzeichnet, dass** die zwei Elektroden (22a, 22b) als Interdigitalelektroden ausgebildet sind.

14. Anordnung nach einem der vorangehenden Ansprüche, **dadurch gekennzeichnet, dass** der NFC-Transponder (24) und die Kapazitätsmesseinrichtung (23) im selben Chip angeordnet sind.

15. Anordnung nach einem der vorangehenden Ansprüche, **dadurch gekennzeichnet, dass** die Schirmung (26) mit der Systemmasse der Kapazitätsmesseinrichtung (23) verbunden ist.

## Claims

1. Arrangement (2) for determining the moisture content of an object (1), comprising an electrically insulating carrier layer (21), on which the following entities are applied
- at least two electrodes (22a, 22b) that are arranged on a first side of the carrier layer (21),
- a digital capacitance measuring device (23), to which the electrodes (22a, 22b) are connected,
- an NFC transponder (24) having an antenna (25), to which NFC transponder (24) the measurement results of the capacitance measuring device (23) are fed for forwarding by means of radio via the antenna (25),
- a shield (26) that lies on the side of the carrier layer (21) opposite to the first side,
**characterised in that**
- a strain relief element (29) is provided on the side of the carrier layer (21) opposite the electrodes (22a, 22b), which strain relief element projects from the carrier layer (21) and is arranged within a border region (21a) around the electrodes (22a, 22b), which border region has a width greater than 15% of the longest dimension of the surface region (220) encompassed by the electrodes (22a, 22b) and
- the strain relief element (29) has a thickness of between 0.5mm and 2mm.

2. Arrangement (2) according to claim 1, further comprising an insulation layer (28), which is arranged on the side of the carrier layer (21) opposite the shield (26) and bears upon the electrodes (22a, 22b),
wherein in particular the insulation layer (28) is formed adhesively on the side facing away from the electrodes (22a, 22b).

3. Arrangement (2) according to claim 2, **characterised in that** the insulation layer (28) comprises a film (282) on the side of which that faces the electrodes (22a,22b) is arranged a first connection layer, in particular an adhesive layer (281), which connects, in particular adhesively, the film (282) with the electrodes (22a, 22b) and/or with the carrier layer (21), and
**in that** on the other side of the film (28), which faces away from the electrodes (22a, 22b), a second adhesive layer (283) is present, for adhering to the object (1).

4. Arrangement (2) according to any one of the preceding claims, **characterised in that** the carrier layer (21) and/or the insulation layer (28) have a relative permittivity of less than 20, in particular less than 5.

5. Arrangement (2) according to any one of the preceding claims, **characterised in that** a cover layer (27) is arranged on the side of the carrier layer (21) on which the shield (26) is disposed.

6. Arrangement (2) according to claim 5, **characterised in that** the cover layer (27) is electrically insulating and electrically insulates the shield (26) against external access.

7. Arrangement (2) according to any one of the preceding claims, **characterised in that** the windings of the antenna (25) are applied and arranged as a conductive layer on one side of the carrier layer (21).

8. Arrangement (2) according to any one of the preceding claims, **characterised in that** the carrier layer (21) and/or the insulation layer (28) and/or the cover layer (27) have a flexible construction.

9. Arrangement (2) according to any one of the preceding claims, **characterised in that** a battery is present, which is connected to the capacitance measuring device (23), and in particular the capacitance measuring device (23) is adapted to measure the capacitance between the electrodes (22a, 22b) at predetermined timepoints and to store the capacitance in a data buffer, wherein the NFC transponder (24) is configured to transmit all capacitance measurement values in the data buffer upon request.

10. Arrangement (2) according to any one of the preceding claims, **characterised in that** the NFC transponder (24) supplies the capacitance measuring device (23) with energy stored in an energy buffer, which energy was drawn from the electromagnetic field surrounding the antenna (25).

11. Arrangement according to any one of the preceding claims, **characterised in that** a border (21a) is provided on the carrier layer (21), in particular on all sides, which is free of electrodes (22a, 22b), wherein the width of the border (21a) of the carrier layer (21) measures at least 15%, in particular at least 30% of the longest dimension of the surface region (220) taken by the electrodes (22a, 22b).

12. Arrangement according to any one of the preceding claims, **characterised in that** the arrangement is configured for determining the moisture content of the human skin.

13. Arrangement according to any one of the preceding claims, **characterised in that** the two electrodes (22a, 22b) are configured as interdigitated electrodes.

14. Arrangement according to any one of the preceding claims, **characterised in that** the NFC transponder (24) and the capacitance measuring device (23) are arranged in the same chip.

15. Arrangement according to any one of the preceding claims, **characterised in that** the shield (26) is connected to the system ground of the capacitance measuring device (23).

## Revendications

1. Ensemble (2) pour déterminer l'humidité d'un objet (1) comprenant une couche porteuse isolante de l'électricité (21) sur laquelle les unités suivantes sont appliquées ;
- au moins deux électrodes (22a, 22b) qui sont agencées sur un premier côté de la couche porteuse (21),
- un dispositif de mesure de capacité numérique (23) auquel les électrodes (22a, 22b) sont connectées,
- un transpondeur NFC (24) avec une antenne (25), auquel les résultats de mesure du dispositif de mesure de capacité (23) sont acheminés pour la transmission par radio via l'antenne (25),
- un bouclier (26) qui se trouve sur le côté de la couche porteuse (21) en regard du premier côté,
**caractérisé en ce que** :
- sur le côté de la couche porteuse (21) qui est en regard des électrodes (22a, 22b), il est prévu un élément de décharge (29) qui est distant de la couche porteuse (21) et est agencé au sein d'une zone de bord (21a) autour des électrodes (22a, 22b), dont la largeur est 15 % supérieure à la dimension la plus longue de la zone de surface (220) entourée par les électrodes (22a, 22b) et
- l'élément de décharge (29) présente une épaisseur entre 0,5 mm et 2 mm.

2. Ensemble (2) selon la revendication 1, comprenant en outre une couche d'isolement (28) qui est agencée sur le côté de la couche porteuse (21) qui est en regard du bouclier (26) et s'applique sur les électrodes (22a, 22b),
dans lequel en particulier la couche d'isolement (28) est conçue de manière à être adhésive sur le côté opposé aux électrodes (22a, 22b).

3. Ensemble (2) selon la revendication 2, **caractérisé en ce que** la couche d'isolement (28) présente une feuille (282) sur le côté tourné vers les électrodes (22a, 22b) duquel est agencée une première couche de liaison, en particulier une couche adhésive (281), qui relie la feuille (282), en particulier par voie adhésive, aux électrodes (22a, 22b) et/ou à la couche porteuse (21) et,
sur l'autre côté de la feuille (28), qui est opposé aux électrodes (22a, 22b), une seconde couche adhésive (283) est présente pour être collée à l'objet (1).

4. Ensemble (2) selon l'une quelconque des revendications précédentes, **caractérisé en ce que** la couche porteuse (21) et/ou la couche d'isolement (28) présente(nt) une permittivité relative de moins de 20, en particulier de moins de 5.

5. Ensemble (2) selon l'une quelconque des revendications précédentes, **caractérisé en ce qu'**il est agencé une couche de recouvrement (27) sur le côté de la couche porteuse (21) où se trouve le bouclier (26).

6. Ensemble (2) selon la revendication 5, **caractérisé en ce que** la couche de recouvrement (27) est conçue de manière à être isolante de l'électricité et le bouclier (26) est isolé électriquement des accès de l'extérieur.

7. Ensemble (2) selon l'une quelconque des revendications précédentes, **caractérisé en ce que** les enroulements de l'antenne (25) sont appliqués et agencés sous la forme d'une couche conductrice sur un côté de la couche porteuse (21).

8. Ensemble (2) selon l'une quelconque des revendications précédentes, **caractérisé en ce que** la couche porteuse (21) et/ou la couche d'isolement (28) et/ou la couche de recouvrement (27) présente(nt) une structure souple.

9. Ensemble (2) selon l'une quelconque des revendications précédentes, **caractérisé en ce qu'**il est prévu une batterie qui est connectée au dispositif de mesure de capacité (23) et, en particulier,
le dispositif de mesure de capacité (23) est aménagé de manière à mesurer, à des moments prédéterminés, la capacité entre les électrodes (22a, 22b) et à enregistrer celle-ci dans une mémoire intermédiaire de données, dans lequel le transpondeur NFC (24) est conçu pour retransmettre toutes les valeurs de mesure de capacité se trouvant dans la mémoire intermédiaire de données à la demande.

10. Ensemble (2) selon l'une quelconque des revendications précédentes, **caractérisé en ce que** le transpondeur NFC (24) alimente le dispositif de mesure de capacité (23) en énergie enregistrée dans une mémoire intermédiaire d'énergie, qui a été prélevée du champ électromagnétique entourant l'antenne (25).

11. Ensemble selon l'une quelconque des revendications précédentes, **caractérisé en ce qu'**il est prévu sur la couche porteuse (21), en particulier sur tous les côtés, un bord (21a) qui est exempt d'électrodes (22a, 22b), dans lequel la largeur du bord (21a) de la couche porteuse (21) mesure en l'occurrence au moins 15 %, en particulier au moins 30 % de la dimension la plus longue de la zone de surface (220) sollicitée par les électrodes (22a, 22b).

12. Ensemble selon l'une quelconque des revendications précédentes, **caractérisé en ce que** l'ensemble est conçu pour déterminer l'humidité de la peau humaine.

13. Ensemble selon l'une quelconque des revendications précédentes, **caractérisé en ce que** les deux électrodes (22a, 22b) sont conçues sous la forme d'électrodes interdigitales.

14. Ensemble selon l'une quelconque des revendications précédentes, **caractérisé en ce que** le transpondeur NFC (24) et le dispositif de mesure de capacité (23) sont agencés dans la même puce.

15. Ensemble selon l'une quelconque des revendications précédentes, **caractérisé en ce que** le bouclier (26) est relié à la masse systémique du dispositif de mesure de capacité (23).
